# EUROPEAN PATENT SPECIFICATION

(11) **EP 3 033 144 B1**
(45) Date of publication and mention of the grant of the patent: **31.05.2023**
(21) Application number: 14752532.3
(22) Date of filing: 05.08.2014
(51) Int. Cl.: A61N 1/375

(54) **LEADLESS CARDIAC PACEMAKER WITH DELIVERY AND/OR RETRIEVAL FEATURES**
LEITUNGSLOSER HERZSCHRITTMACHER MIT ABGABE- UND/ODER ENTNAHMEFUNKTION
STIMULATEUR CARDIAQUE SANS FIL COMPORTANT DES FONCTIONS D'ADMINISTRATION ET/OU DE RÉCUPÉRATION

(30) Priority: 16.08.2013 US 201361866640 P; 16.08.2013 US 201361866644 P
(43) Date of publication of application: 22.06.2016
(73) Proprietor: Cardiac Pacemakers, Inc., St. Paul, Minnesota 55112 (US)
(72) Inventor: SCHMIDT, Brian L., White Bear Lake Minnesota 55110 (US); HAASL, Benjamin J., Forest Lake Minnesota 55025 (US); MAILE, Keith R., New Brighton Minnesota 55112 (US); SACHS, Dana, Pine City Minnesota 55063 (US)
(74) Representative: Pfenning, Meinig & Partner mbB
(86) International application number: PCT/US2014/049761
(87) International publication number: WO 2015/023473

(56) References cited:
- WO-A1-2012/082735
- WO-A2-2013/067496
- US-A1- 2013 053 921

## Description

### TECHNICAL FIELD

The disclosure is directed to implantable cardiac devices. More particularly, the disclosure is directed to leadless cardiac stimulators or pacemakers including delivery and/or retrieval features.

### BACKGROUND

Cardiac pacemakers provide electrical stimulation to heart tissue to cause the heart to contract and thus pump blood through the vascular system. Conventional pacemakers typically include an electrical lead that extends from a pulse generator implanted subcutaneously or sub-muscularly to an electrode positioned adjacent the inside or outside wall of the cardiac chamber. As an alternative to conventional pacemakers, self-contained or leadless cardiac pacemakers have been proposed. Leadless cardiac pacemakers are small capsules typically fixed to an intracardiac implant site in a cardiac chamber with a fixation mechanism engaging the intracardiac tissue. The small capsule typically includes bipolar pacing/sensing electrodes, a power source (e.g. a battery), and associated electrical circuitry for controlling the pacing/sensing electrodes, and thus provide electrical stimulation to heart tissue and/or sense a physiological condition.

Accordingly, there it is desirable to provide alternative structures to facilitate delivering leadless cardiac pacemakers to an implantation site in a heart chamber and/or retrieving leadless cardiac pacemakers from an implantation site in a heart chamber Document WO-A-2012/082735 discloses the most relevant prior art.

### SUMMARY

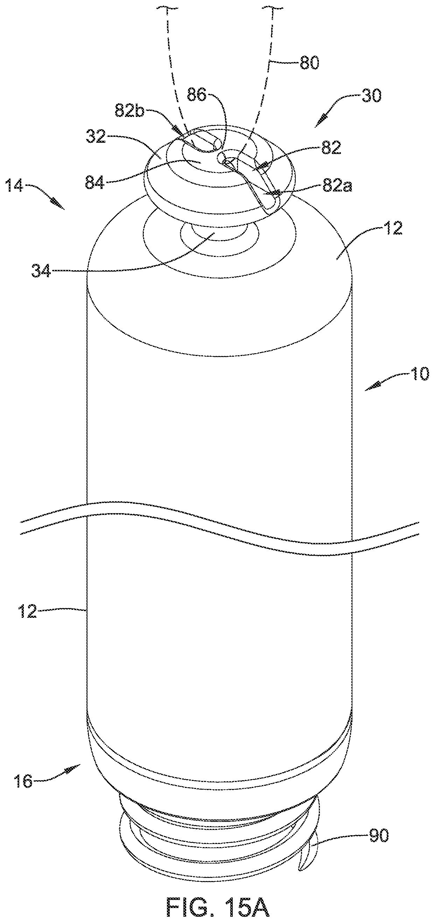

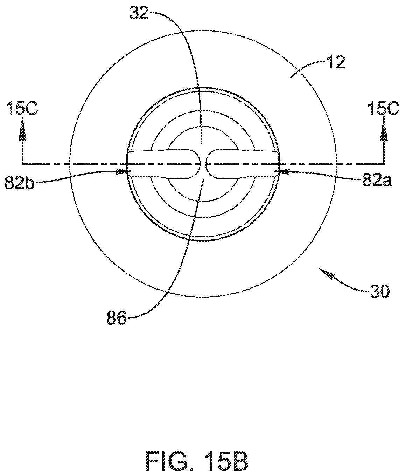

The invention is defined in claim 1. The invention is illustrated by the embodiments shown in figures 15A-15C, 16A-16C, 17A-17C, 18A-18D and the passages of the description relating to these embodiments. The embodiments illustrated by the other figures are not part of the invention.

The above summary of some example embodiments is not intended to describe each disclosed embodiment or every implementation of the aspects of the disclosure.

### BRIEF DESCRIPTION OF THE DRAWINGS

The aspects of the disclosure may be more completely understood in consideration of the following detailed description of various embodiments in connection with the accompanying drawings, in which:
FIG. 1 illustrates an exemplary implantable device implanted in a chamber of a heart;
FIG. 2 illustrates an exemplary retrieval device capturing an implantable device during a retrieval procedure;
FIG. 3 illustrates another exemplary retrieval device capturing an implantable device during a retrieval procedure;
FIGS. 4A-4C illustrate an exemplary docking member of an implantable device;
FIGS. 5A-5C illustrate another exemplary docking member of an implantable device;
FIGS. 6A-6C illustrate another exemplary docking member of an implantable device;
FIGS. 7A-7C illustrate another exemplary docking member of an implantable device;
FIGS. 8A-8C illustrate another exemplary docking member of an implantable device;
FIGS. 9A-9C illustrate another exemplary docking member of an implantable device;
FIGS. 10A-10C illustrate another exemplary docking member of an implantable device;
FIGS. 11A-11C illustrate another exemplary docking member of an implantable device;
FIGS. 12A-12C illustrate another exemplary docking member of an implantable device;
FIGS. 13A-13C illustrate another exemplary docking member of an implantable device;
FIGS. 14A-14C illustrate another exemplary docking member of an implantable device;
FIGS. 15A-15C illustrate another exemplary docking member of an implantable device;
FIGS. 16A-16C illustrate another exemplary docking member of an implantable device;
FIG. 17A illustrates an exemplary delivery device for delivering an implantable device;
FIG. 17B illustrates the delivery device of FIG. 17A in engagement with the docking member of the implantable device of FIGS. 15A-15C;
FIG. 17C illustrates the delivery device of FIG. 17A in engagement with the docking member of the implantable device of FIGS. 16A-16C; and
FIGS. 18A-18D illustrate alternative embodiments of a driver mechanism for a rotational driving instrument.

While the aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the disclosure.

### DETAILED DESCRIPTION

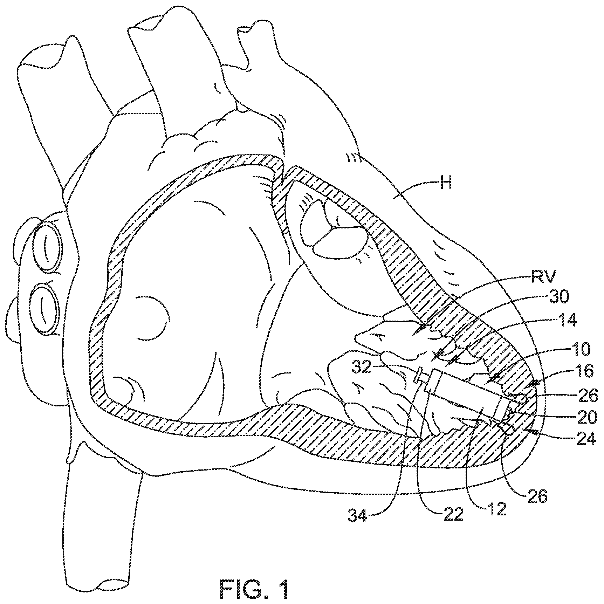

Referring to FIG. 1, an exemplary implantable leadless cardiac pacing device 10 (e.g., a leadless pacemaker) is illustrated implanted in a chamber of a heart H, such as the apex of the right ventricle RV. The implantable device 10 may include a shell or housing 12 having a proximal end 14 and a distal end 16. The implantable device 10 may include a first electrode 20 positioned proximate the distal end 16 of the housing 12 and a second electrode 22 positioned proximate the proximal end 14 of the housing 12. The electrodes 20, 22 may be sensing and/or pacing electrodes to provide electro-therapy and/or sensing capabilities. The first electrode 20 may be configured to be positioned against or otherwise contact the cardiac tissue of the heart H while the second electrode 22 may be spaced away from the first electrode 20, and thus spaced away from the cardiac tissue.

The implantable device 10 may include a pulse generator (e.g., electrical circuitry) and a power source (e.g., a battery) within the housing 12 to provide electrical signals to the electrodes 20, 22 and thus control the pacing/sensing electrodes 20, 22. Electrical communication between pulse generator and the electrodes 20, 22 may provide electrical stimulation to heart tissue and/or sense a physiological condition.

The implantable device 10 may include a fixation mechanism 24 proximate the distal end 16 of the housing 12 configured to attach the implantable device 10 to a tissue wall of the heart H, or otherwise anchor the implantable device 10 to the anatomy of the patient. As shown in FIG. 1, in some instances, the fixation mechanism 24 may include one or more, or a plurality of hooks 26 anchored into the cardiac tissue of the heart H to attach the implantable device 10 to a tissue wall. In other instances, the fixation mechanism 24 may include one or more, or a plurality of passive tines, configured to entangle with trabeculae within the chamber of the heart H and/or a helical fixation anchor configured to be screwed into a tissue wall to anchor the implantable device 10 to the heart H.

The implantable device 10 may include a docking member 30 proximate the proximal end 14 of the housing 12 configured to facilitate delivery and/or retrieval of the implantable device 10. For example, the docking member 30 may extend from the proximal end 14 of the housing 12 along a longitudinal axis of the housing 12. The docking member 30 may include a head portion 32 and a neck portion 34 extending between the housing 12 and the head portion 32. The head portion 32 may be an enlarged portion relative to the neck portion 34. For example, the head portion 32 may have a radial dimension from the longitudinal axis of the implantable device 10 which is greater than a radial dimension of the neck portion from the longitudinal axis of the implantable device 10. The docking member 30 may be configured to facilitate delivery of the implantable device 10 to the intracardiac site and/or retrieval of the implantable device 10 from the intracardiac site. Some exemplary embodiments of the docking member 30 are described in further detail herein.

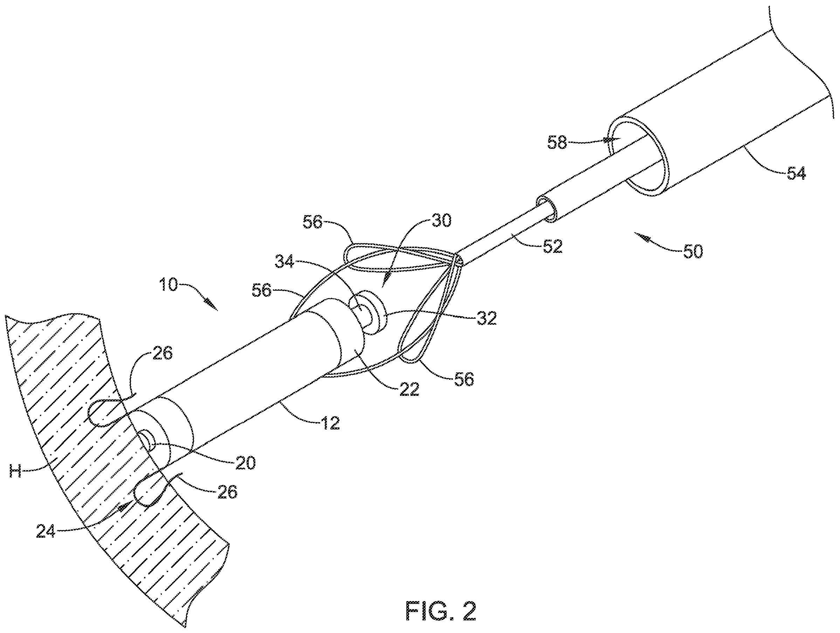

If it is desired to retrieve the implantable device 10 from the heart H, a retrieval device 50 may be advanced into the chamber of the heart H to capture the implantable device 10 and remove the implantable device 10 from the heart H. One exemplary retrieval device 50 is illustrated in FIG. 2. The retrieval device 50 may include a snare 52 advanceable from a lumen 58 of a retrieval catheter 54. The snare 52 may include one or more, or a plurality of loops 56 extending from a distal end of the snare 52 configured to engage the docking member 30 of the implantable device 10. The snare 52 shown in FIG. 2 includes three loops 56 formed by elongate filaments extending from the shaft of the snare 52. Once the loop(s) 56 of the snare 52 has captured the docking member 30, the snare 52 may be actuated proximally relative to the retrieval catheter 54 to pull the implantable device 10 into the lumen 58 of the retrieval catheter 54. The enlarged size of the head portion 32 relative to the neck portion 34 may permit the loop 56 of the snare 52 to encircle the neck portion 34 below (i.e., distal of) the head portion 32 and retain the loop 56 around the docking member 30 as the snare 52 is pulled proximally. As the implantable device 10 is pulled into the retrieval catheter 54, the fixation mechanism 24 may disengage from the heart tissue to detach the implantable device 10 from the heart wall. For example, the hooks 26 may elongate as the implantable device 10 is drawn proximally into the lumen 58 of the retrieval catheter 54. Thereafter, the retrieval device 50, with the implantable device 10 captured in the lumen of the retrieval catheter 54 with the snare 52, may be withdrawn from the heart H.

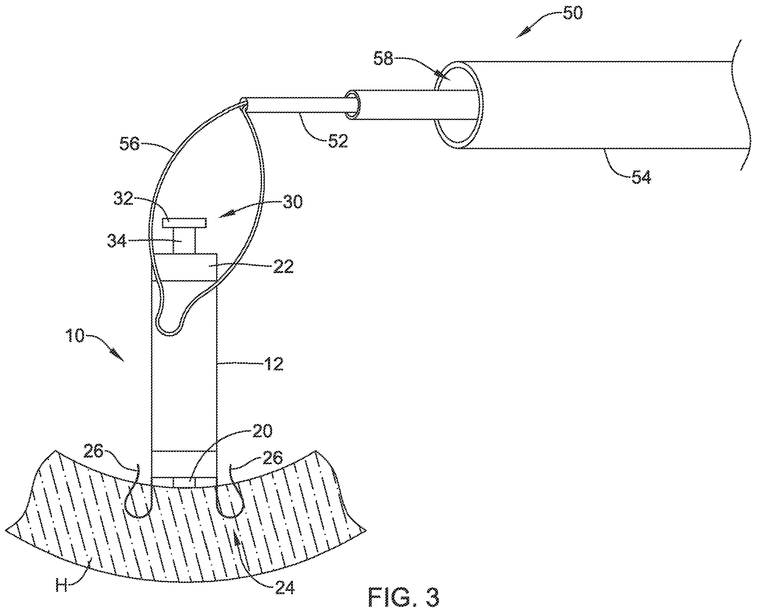

Another exemplary retrieval device 50 is illustrated in FIG. 3. Similar to FIG. 2, the retrieval device 50 may include a snare 52 advanceable from a lumen 58 of a retrieval catheter 54. The snare 52 may include one or more, or a plurality of loops 56 extending from a distal end of the snare 52 configured to engage the docking member 30 of the implantable device 10. The snare 52 shown in FIG. 3 includes a single loop 56 formed by an elongate filament extending from the shaft of the snare 52. Once the loop 56 of the snare 52 has captured the docking member 30, the snare 52 may be actuated proximally relative to the retrieval catheter 54 to pull the implantable device 10 into the lumen 58 of the retrieval catheter 54. The enlarged size of the head portion 32 relative to the neck portion 34 may permit the loop 56 of the snare 52 to encircle the neck portion 34 below (i.e., distal of) the head portion 32 and retain the loop 56 around the docking member 30 as the snare 52 is pulled proximally. As the implantable device 10 is pulled into the retrieval catheter 54, the fixation mechanism 30 may disengage from the heart tissue to detach the implantable device 10 from the heart wall. For example, the hooks 26 may elongate as the implantable device 10 is drawn proximally into the lumen 58 of the retrieval catheter 54. Thereafter, the retrieval device 50, with the implantable device 10 captured in the lumen of the retrieval catheter 54 with the snare 52, may be withdrawn from the heart H.

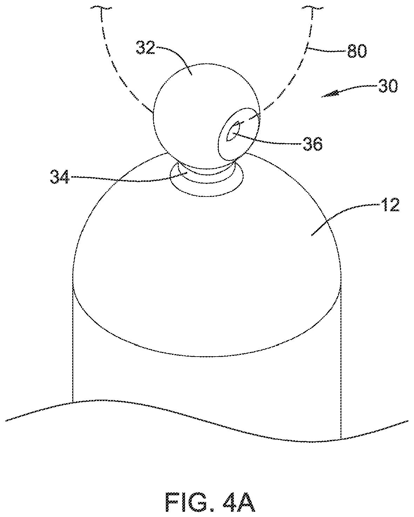

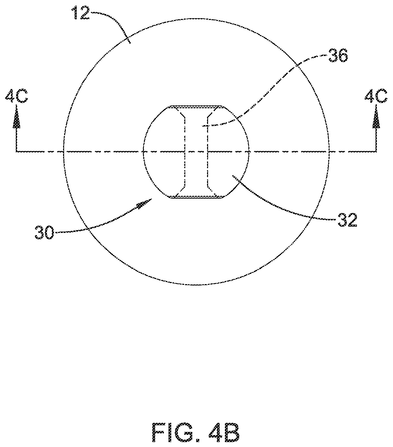

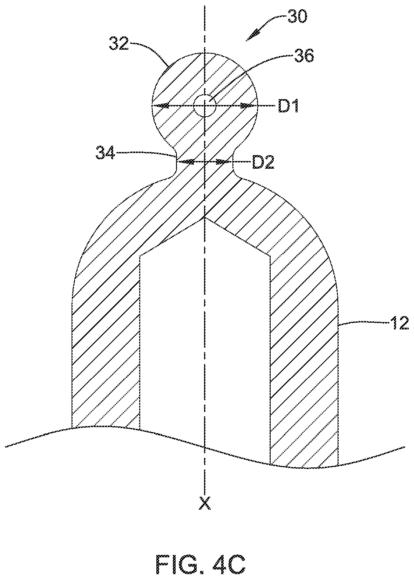

FIGS. 4A-4C illustrate one exemplary docking member 30 located at the proximal end 14 of the implantable device 10. The docking member 30 shown in FIGS. 4A-4C may include a head portion 32 and a neck portion 34 extending between the housing 12 and the head portion 32. The head portion 32 may be a generally spherically shaped ball, having a diameter D1 greater than the diameter D2of the neck portion 34. The docking member 30 may also include a passage 36 extending through a portion of the docking member 30 to receive a tether 80 (shown in phantom lines) which will be further described later herein. For example, the passage 36 may extend through the head portion 32 from a first side to a second side of the head portion 32. The spherical shape of the head portion 32 may provide an atraumatic profile, inhibiting tissue growth or entanglement around the docking member 30.

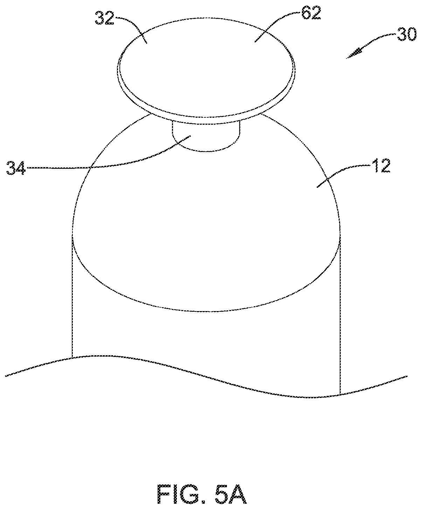

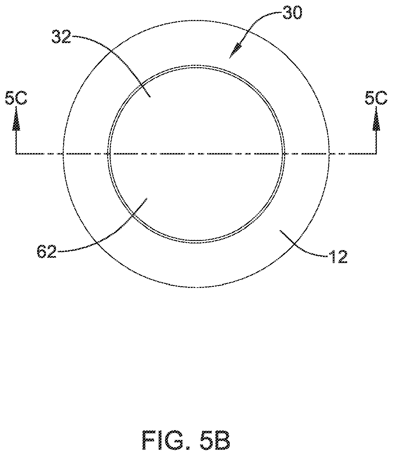

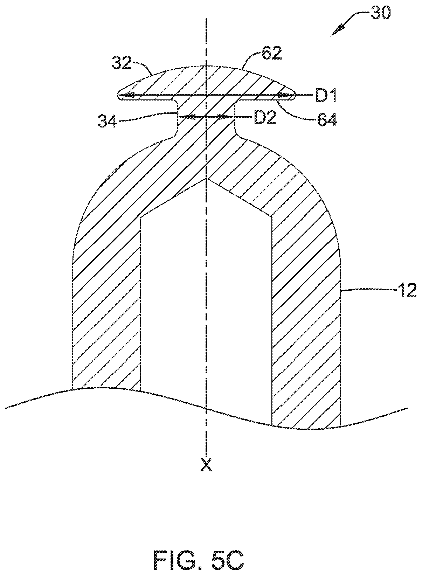

FIGS. 5A-5C illustrate another exemplary docking member 30 located at the proximal end 14 of the implantable device 10. The docking member 30 shown in FIGS. 5A-5C may include a head portion 32 and a neck portion 34 extending between the housing 12 and the head portion 32. The head portion 32 may be a generally disc shaped element having an upper surface 62 and an opposing lower surface 64. In some instances, the upper surface 62 and/or the lower surface 64 may be a spherically convex surface while in other instances the upper surface 62 and/or the lower surface 64 may be a spherically concave surface or a planar surface, for example. The head portion 32 may have a diameter D1 greater than the diameter D2 of the neck portion 34. Although not shown, the docking member 30 may also include a passage extending through a portion of the docking member 30 to receive a tether (described later herein). For example, a passage may extend through the head portion 32 from a first side to a second side of the head portion 32, or a passage may extend through the neck portion 34 from a first side to a second side of the neck portion 34. The shape of the head portion 32 may provide an atraumatic profile, inhibiting tissue growth or entanglement around the docking member 30.

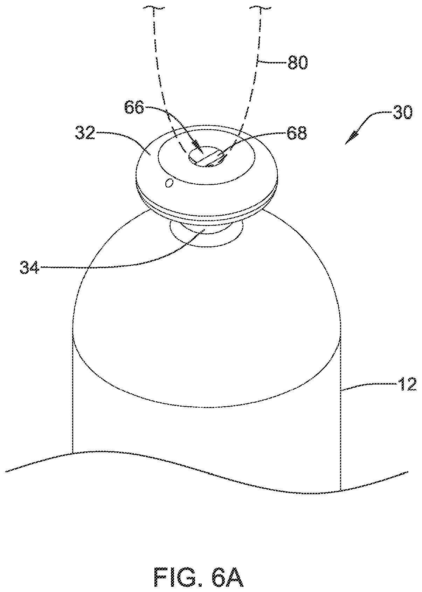

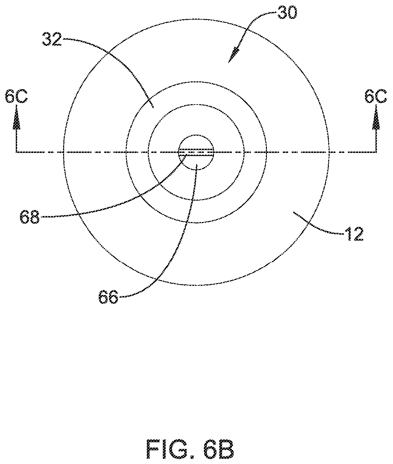

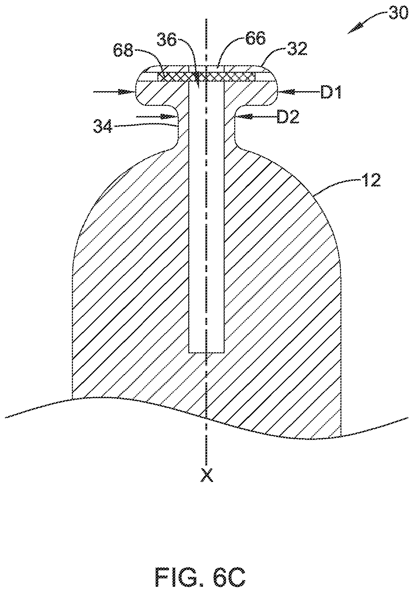

FIGS. 6A-6C illustrate another exemplary docking member 30 located at the proximal end 14 of the implantable device 10. The docking member 30 shown in FIGS. 6A-6C may include a head portion 32 and a neck portion 34 extending between the housing 12 and the head portion 32. The head portion 32 may be generally knob shaped, having a diameter D 1 greater than the diameter D2 of the neck portion 34. The docking member 30 may also include a passage 36 extending through a portion of the docking member 30 to receive a tether 80 (shown in phantom) further described later herein. For example, the head portion 32 may include a central opening 66 extending into the head portion 32. A pin 68 may extend into or across the opening 66. For example, as shown in FIG. 6C the pin 68 may extend from a first side to a second side of the opening 66. In other instances, the pin 68 may extend into the opening 66 from a first side toward a second side of the opening 66, but not entirely across the opening 66 to the second side. The passage 36 may extend under the pin 68 such that the tether may be passed around the pin 68. The shape of the head portion 32 may provide an atraumatic profile, inhibiting tissue growth or entanglement around the docking member 30.

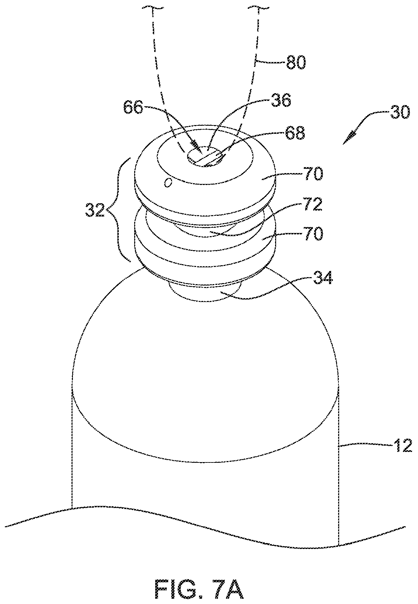

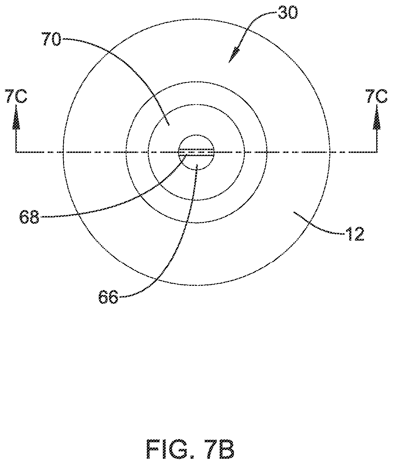

FIGS. 7A-7C illustrate another exemplary docking member 30 located at the proximal end 14 of the implantable device 10. The docking member 30 shown in FIGS. 7A-7C may include a head portion 32 and a neck portion 34 extending between the housing 12 and the head portion 32. The head portion 32 may include a plurality of enlarged portions 70 spaced apart by a reduced diameter necked portion 72. In some instances, the enlarged portions 70 may be generally knob shaped, having a diameter D1 greater than the diameter D3 of the necked portion between the enlarged portions 70 and the diameter D2 of the neck portion 34. Multiple enlarged portions 70 may facilitate engaging the docking member 30 with the loop(s) 56 of the snare 52 during retrieval of the implantable device 10. The shape of the head portion 32 may provide an atraumatic profile, inhibiting tissue growth or entanglement around the docking member 30.

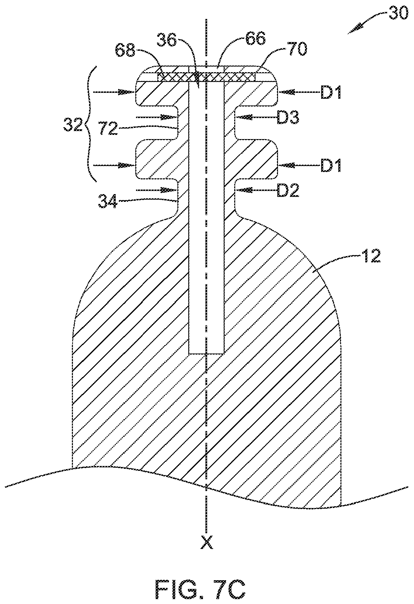

The docking member 30 may also include a passage 36 extending through a portion of the docking member 30 to receive a tether (described later herein). For example, the head portion 32 may include a central opening 66 extending into the head portion 32. A pin 68 may extend into or across the opening 66. For example, as shown in FIG. 7C the pin 68 may extend from a first side to a second side of the opening 66. In other instances, the pin 68 may extend into the opening 66 from a first side toward a second side of the opening 66, but not entirely across the opening 66 to the second side. The passage 36 may extend under the pin 68 such that the tether may be passed around the pin 68.

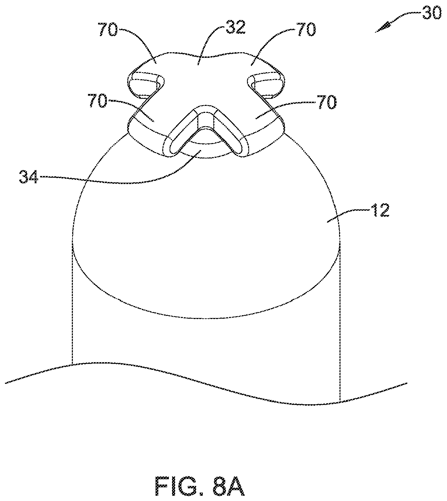

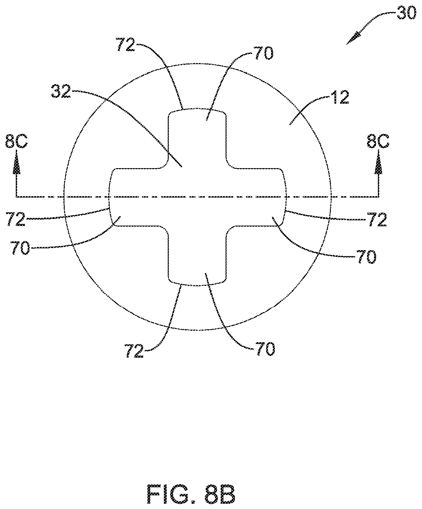

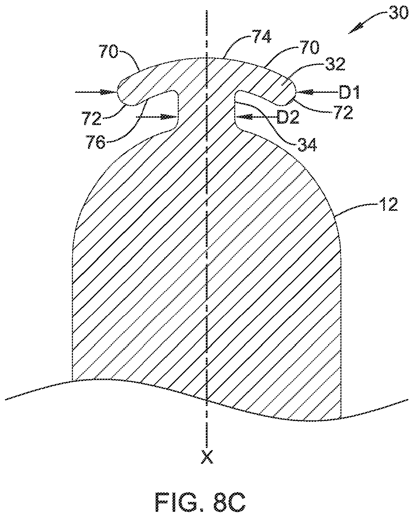

FIGS. 8A-8C illustrate another exemplary docking member 30 located at the proximal end 14 of the implantable device 10. The docking member 30 shown in FIGS. 8A-8C may include a head portion 32 and a neck portion 34 extending between the housing 12 and the head portion 32. The head portion 32 may have a diameter D1 greater than the diameter D2 of the neck portion 34. The head portion 32 may include a plurality of spokes 70 extending radially from the longitudinal axis of the implantable device 10, with spaces defined between adjacent spokes 70. The spokes 70 may be symmetrically or asymmetrically arranged around the longitudinal axis X. For example, the head portion 32 may include four spokes 70 uniformly arranged around the longitudinal axis X about 90 degrees apart. As shown in FIG. 8X, the free ends 72 of the radially extending spokes 70 may angle (e.g, curve) toward the distal end 16 of the implantable device 10, in some instances. For example, an upper surface 74 and/or a lower surface 76 of the spokes 70 may extend at an oblique angle to the longitudinal axis X of the implantable device 10 toward the distal end 16, such that the free ends 72 of the spokes 70 are positioned closer to the distal end 16 of the implantable device 10 than the base portion of the spokes 70 proximate the central longitudinal axis X. The configuration and/or arrangement of the spokes 70 may facilitate retention of the loop 56 of the snare 52 in engagement of the docking member 30 during retrieval of the implantable device 10. For example, the loop 56 may encircle one or more of the spokes 70 in addition to or instead of the neck portion 34.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

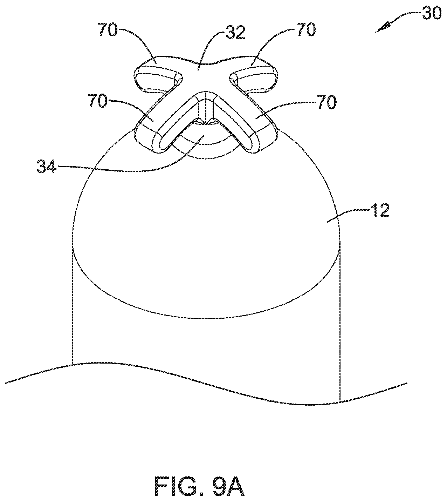

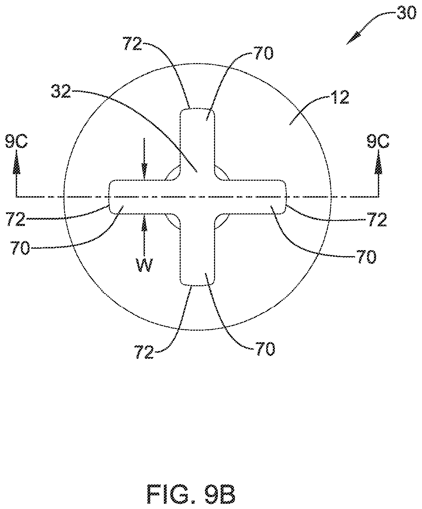

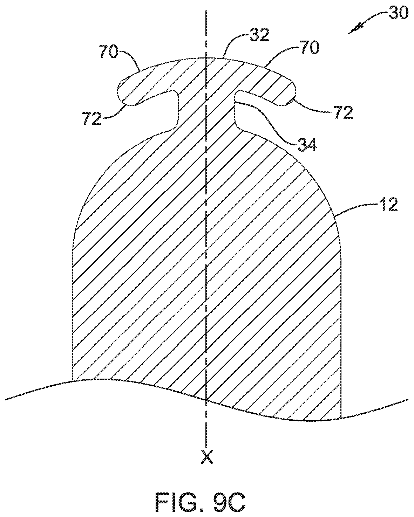

FIGS. 9A-9C illustrate another exemplary docking member 30 located at the proximal end 14 of the implantable device 10, similar to the docking member 30 illustrated in FIGS. 8A-8C. The docking member 30 shown in FIGS. 9A-9C may similarly include a plurality of spokes 70 extending radially from the longitudinal axis of the implantable device 10, with spaces defined between adjacent spokes 70, except the spokes 70 shown in FIGS. 9A-9C may have a width W less than the width of the spokes shown in FIGS. 8A-8C.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

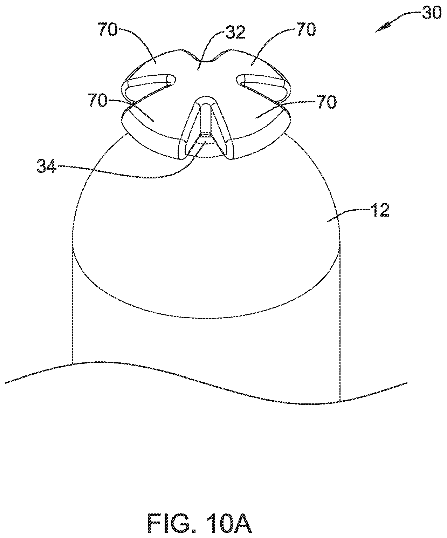

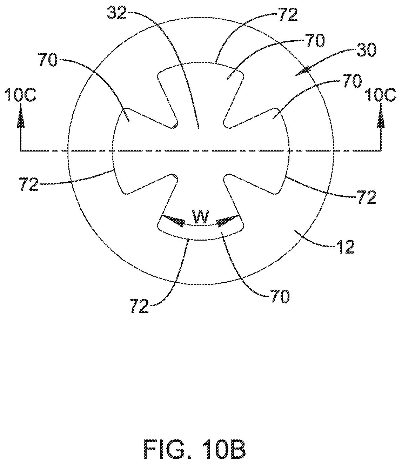

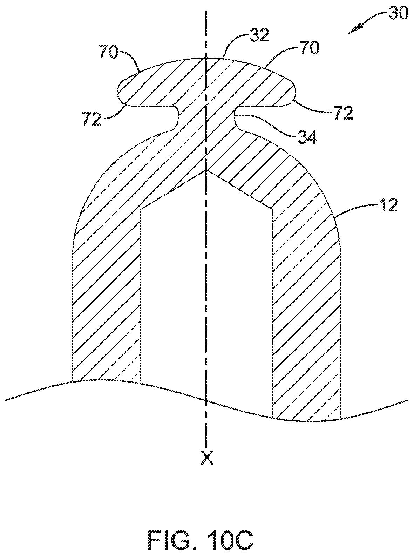

FIGS. 10A-10C illustrate another exemplary docking member 30 located at the proximal end 14 of the implantable device 10, similar to the docking member 30 illustrated in FIGS. 8A-8C. The docking member 30 shown in FIGS. 10A-10C may similarly include a plurality of spokes 70 extending radially from the longitudinal axis of the implantable device 10, with spaces defined between adjacent spokes 70. The width W of the spokes 70 shown in FIGS. 10A-10C may increase from the base portion toward the free ends 72 of the spokes. Thus, in instances in which the loop 56 of the snare 52 encircles one or more of the spokes 70, the enlarged free end 72 of the spokes 70 may prevent the loop 56 of the snare 52 from slipping off the spoke(s) 70 during retrieval of the implantable device 10.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

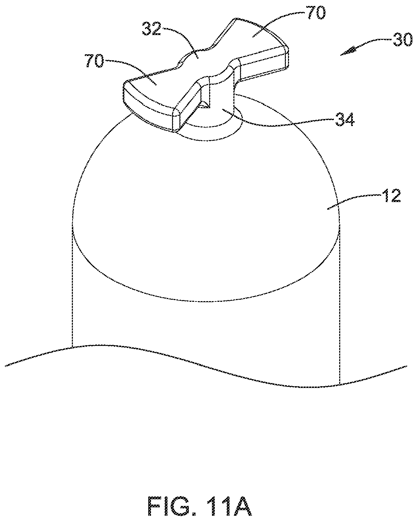

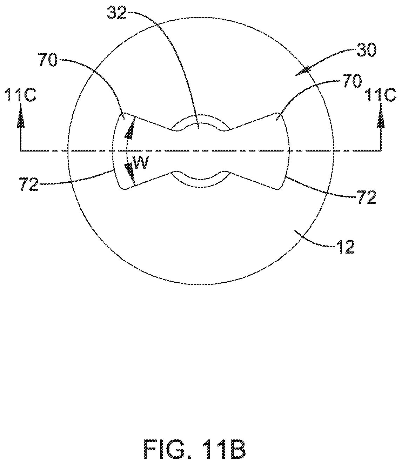

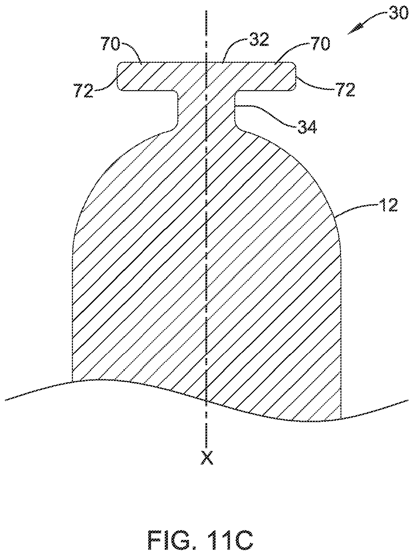

FIGS. 11A-11C illustrate another exemplary docking member 30 located at the proximal end 14 of the implantable device 10, similar to the docking member 30 illustrated in FIGS. 8A-8C. The docking member 30 shown in FIGS. 11A-11C may include a pair of spokes 70 extending radially from the longitudinal axis of the implantable device 10 in opposite directions. Similar to the spokes 70 shown in FIGS. 10A-10C, the width W of the spokes 70 shown in FIGS. 11A-11C may increase from the base portion toward the free ends 72 of the spokes. Thus, in instances in which the loop 56 of the snare 52 encircles one or more of the spokes 70, the enlarged free end 72 of the spokes 70 may prevent the loop 56 of the snare 52 from slipping off the spoke(s) 70 during retrieval of the implantable device 10.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

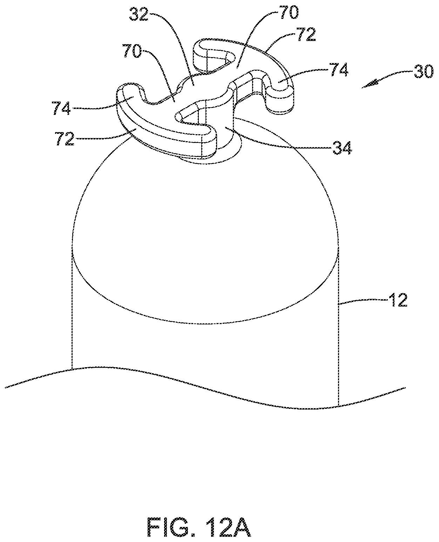

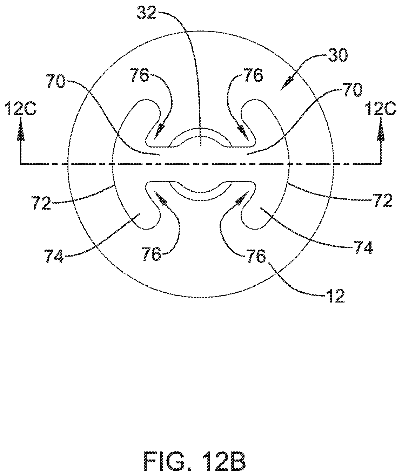

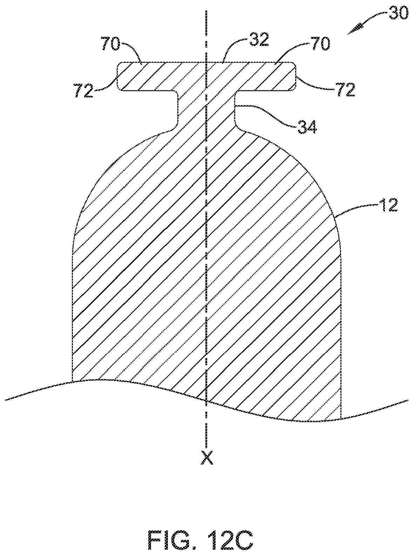

FIGS. 12A-12C illustrate another exemplary docking member 30 located at the proximal end 14 of the implantable device 10, similar to the docking member 30 illustrated in FIGS. 11A-11C. The docking member 30 shown in FIGS. 12A-12C may include a pair of spokes 70 extending radially from the longitudinal axis of the implantable device 10 in opposite directions. The free ends 72 of the spokes 70 may include a crossing member 74 extending transverse to the radial direction of the spokes 70. In some instances, the crossing member 74 may extend in opposite directions from the body of the spoke 70, or the crossing member 74 may extend in one transverse direction from the body of the spoke 70. For example, in some instances, the spokes 70 may have a T shape, a mushroom shape, an L shape, a V shape, or other desired shape. In some instances, the crossing member 74 may form an undercut 76 with the body of the spoke 70. The loop 56 of the snare 52 may be engaged in the undercuts 76 as the loop 56 is tightened around the spoke 70. In instances in which the loop 56 of the snare 52 encircles one or more of the spokes 70, the crossing member 74 at the free end 72 of the spokes 70 may prevent the loop 56 of the snare 52 from slipping off the spoke(s) 70 during retrieval of the implantable device 10.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

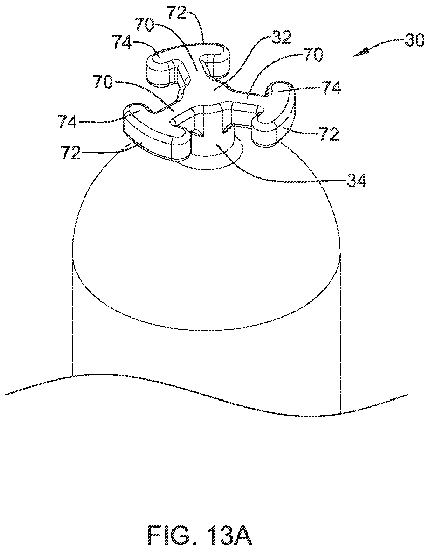

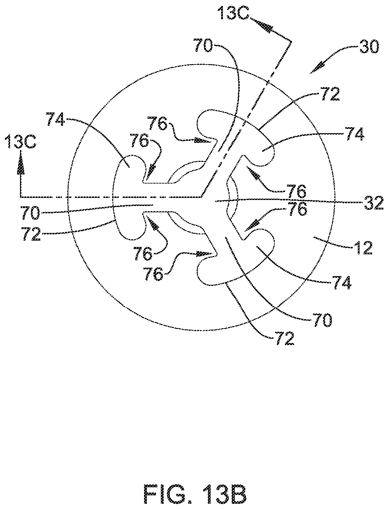

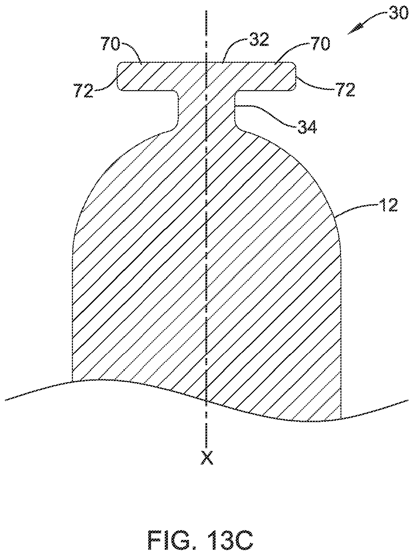

FIGS. 13A-13C illustrate another exemplary docking member 30 located at the proximal end 14 of the implantable device 10, similar to the docking member 30 illustrated in FIGS. 12A-12C. The docking member 30 shown in FIGS. 13A-13C may include a plurality of spokes 70 extending radially from the longitudinal axis of the implantable device 10, with spaces defined between adjacent spokes 70. The spokes 70 may be symmetrically or asymmetrically arranged around the longitudinal axis. For example, the head portion 32 may include three spokes 70 uniformly arranged around the longitudinal axis about 120 degrees apart. Similar to the spokes illustrated in FIGS. 12A-12C, the free ends 72 of the spokes 70 may include a crossing member 74 extending transverse to the radial direction of the spokes 70. In some instances, the crossing member 74 may extend in opposite directions from the body of the spoke 70, or the crossing member 74 may extend in one transverse direction from the body of the spoke 70. For example, in some instances, the spokes 70 may have a T shape, a mushroom shape, an L shape, a V shape, or other desired shape. In some instances, the crossing member 74 may form an undercut 76 with the body of the spoke 70. The loop 56 of the snare 52 may be engaged in the undercuts 76 as the loop 56 is tightened around the spoke 70. In instances in which the loop 56 of the snare 52 encircles one or more of the spokes 70, the crossing member 74 at the free end 72 of the spokes 70 may prevent the loop 56 of the snare 52 from slipping off the spoke(s) 70 during retrieval of the implantable device 10.

Although not shown, the docking member 30 may also include a passage extending through a portion of the docking member 30 to receive a tether (described later herein). For example, a passage may extend through the one or more of the spokes 70 from a first side to a second side of the spoke 70, or a passage may extend through the neck portion 34 from a first side to a second side of the neck portion 34.

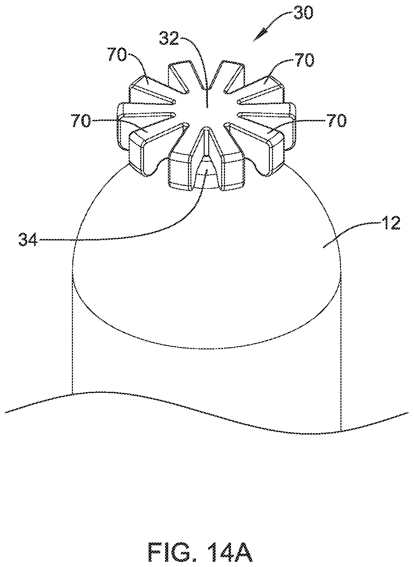

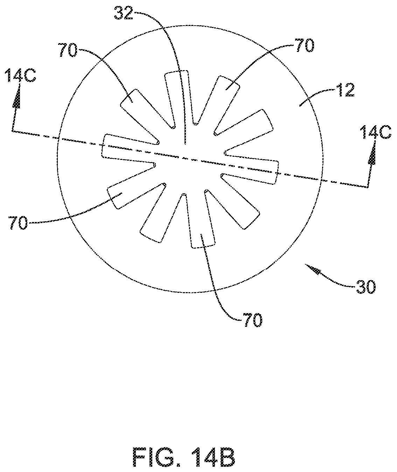

FIGS. 14A-14C illustrate another exemplary docking member 30 located at the proximal end 14 of the implantable device 10, similar to the docking member 30 illustrated in FIGS. 8A-8C. The docking member 30 shown in FIGS. 14A-14C may similarly include a plurality of spokes 70 extending radially from the longitudinal axis of the implantable device 10, with spaces defined between adjacent spokes 70. The docking member 30 may include any number of radially extending spokes 70 symmetrically or asymmetrically arranged around the longitudinal axis of the implantable device 10. For example, the docking member 30 may include 3, 4, 5, 6, 7, 8, 9, 10, 11, 12 or more radially extending spokes 70 in some instances.

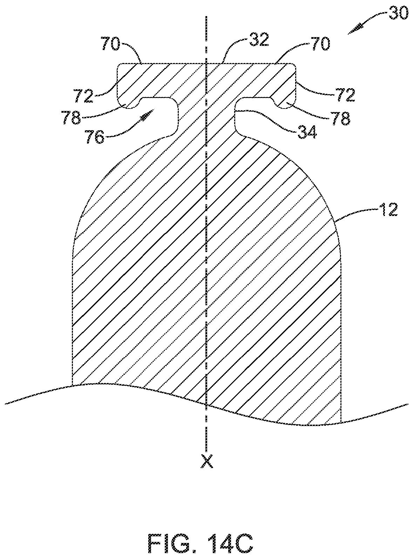

As shown in FIG. 14C, the free ends 72 of the spokes 70 may include a distally projecting lip 78 relative to the body portion of the spokes 70. The distally projecting lips 78 may prevent the loop 56 of the snare 52 from slipping off the spoke(s) 70 during retrieval of the implantable device 10.

The configuration and/or arrangement of the spokes 70 may facilitate retention of the loop 56 of the snare 52 in engagement of the docking member 30 during retrieval of the implantable device 10. For example, the loop 56 may encircle one or more of the spokes 70 in addition to or instead of the neck portion 34.

In some instances it may be desirable to apply rotational motion to the implantable device 10 during delivery and/or retrieval of the implantable device 10. For example, in some embodiments such as the embodiment shown in FIG. 15A, the implantable device 10 may include a helical fixation anchor 90 at the distal end 16 of the housing 12 configured to be screwed into a tissue wall through rotational motion of the implantable device 10 to anchor the implantable device 10 to the heart H. In such instances, the docking member 30 may include an engagement feature configured to mate with an engagement feature of a delivery device to transfer rotational motion from a rotatable shaft of the delivery device to the implantable device 10.

For example, in the embodiment of FIGS. 15A-15C, the implantable device 10 may include a docking member 30 extending from the proximal end 14 of the housing 12 along a longitudinal axis of the housing 12. The docking member 30 may include a head portion 32 and a neck portion 34 extending between the housing 12 and the head portion 32. The head portion 32 may be an enlarged portion relative to the neck portion 34. For example, the head portion 32 may have a radial dimension from the longitudinal axis of the implantable device 10 which is greater than a radial dimension of the neck portion from the longitudinal axis of the implantable device 10.

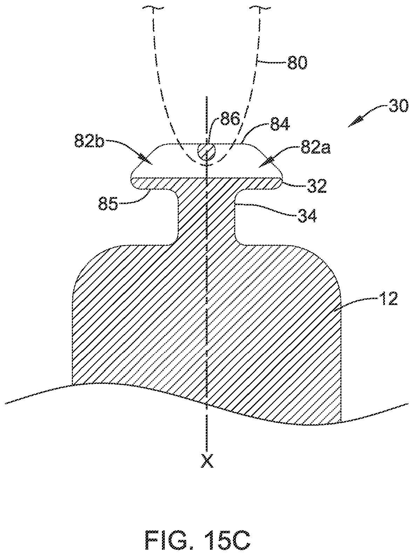

The head portion 32 may include a recess 82 extending into the head portion 32 from a proximal surface 84 of the head portion 32. The recess 82 may be configured to receive a rotational driving instrument therein. For example, the recess 82 may be configured to receive a distal driver mechanism of a rotational driving instrument therein. In some instances, the recess 82 may extend generally perpendicular to the longitudinal axis of the housing 12. In some embodiments, the recess 82 may extend across the head portion 32 from a first side of the head portion 32 to a second side of the head portion 32. As shown in FIG. 15C, the head portion 32 may include a distal surface 85 opposite the proximal surface 84 from which the neck portion 34 extends from. In some instances, the recess 82 may extend into the head portion 32 from the proximal surface 84 toward the distal surface 85, but does not extend to the distal surface 85. However, in other embodiments the recess 82 may extend to the distal surface 85.

The head portion 32 may also include a member 86 extending across the recess 82 dividing the recess 82 into a first recess portion 82a on a first side of the member 86 and a second recess portion 82b on a second side of the member 86. In some instances, the member 86 may extend generally perpendicular to the recess 82 and/or the longitudinal axis of the housing 12. As shown in FIGS. 15A-15C, in some instances the member 86 may be formed as a monolithic portion of the head portion 32 bridging across the recess 82. In other embodiments, however, the member 86 may be a separate component attached to the head portion 32.

A tether 80 may extend through a passage beneath the member 86 defined by the recess 82 during delivery of the implantable device 10. For example, the tether 80 may be attached to the member 86 and extend proximally from the member 86 along an elongate shaft of a delivery device to a location accessible by a physician during implantation of the implantable device 10. Once the implantable device 10 has been properly implanted in the heart H, the tether 80 may be detached from the member 86 and withdrawn from the patient.

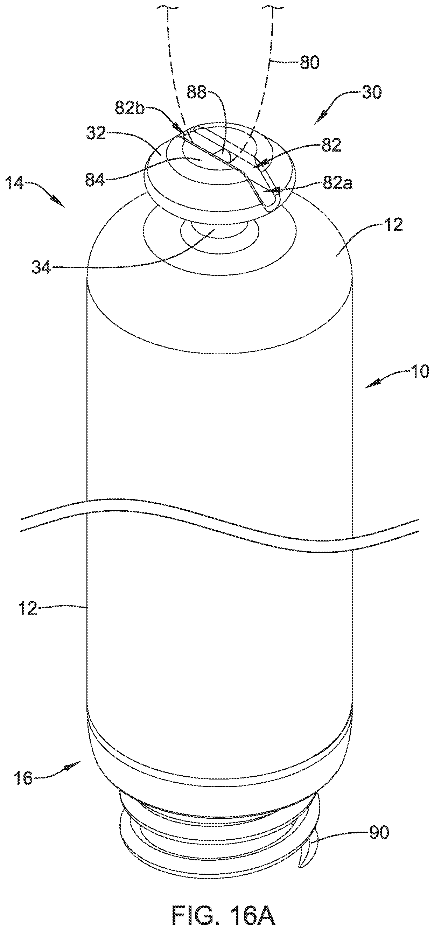

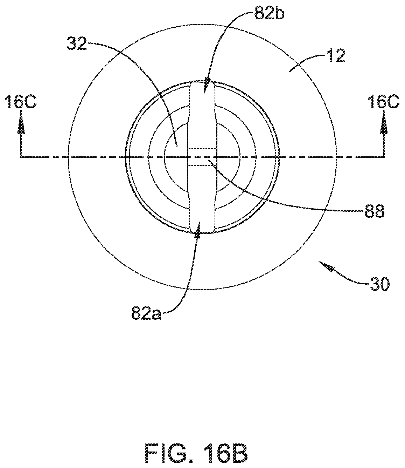

FIGS. 16A-16C illustrate another implantable device 10 including a helical fixation anchor 90 at the distal end 16 of the housing 12 configured to be screwed into a tissue wall through rotational motion of the implantable device 10 to anchor the implantable device 10 to the heart H. Similar to the embodiment of FIGS. 15A-15C, the docking member 30 may include an engagement feature configured to mate with an engagement feature of a delivery device to transfer rotational motion from a rotatable shaft of the delivery device to the implantable device 10.

For example, in the embodiment of FIGS. 16A-16C, the implantable device 10 may include a docking member 30 extending from the proximal end 14 of the housing 12 along a longitudinal axis of the housing 12. The docking member 30 may include a head portion 32 and a neck portion 34 extending between the housing 12 and the head portion 32. The head portion 32 may be an enlarged portion relative to the neck portion 34. For example, the head portion 32 may have a radial dimension from the longitudinal axis of the implantable device 10 which is greater than a radial dimension of the neck portion from the longitudinal axis of the implantable device 10.

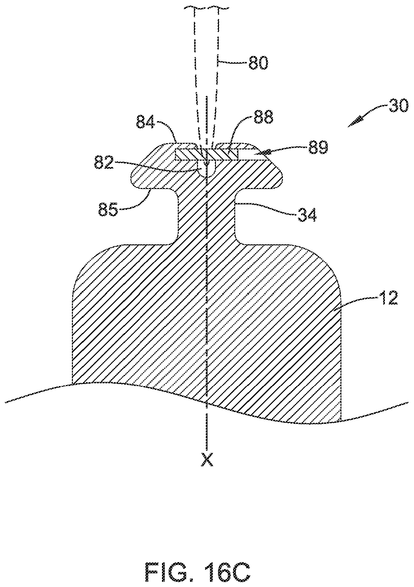

The head portion 32 may include a recess 82 extending into the head portion 32 from a proximal surface 84 of the head portion 32. The recess 82 may be configured to receive a rotational driving instrument therein. For example, the recess 82 may be configured to receive a distal driver mechanism of a rotational driving instrument therein. In some instances, the recess 82 may extend generally perpendicular to the longitudinal axis of the housing 12. In some embodiments, the recess 82 may extend across the head portion 32 from a first side of the head portion 32 to a second side of the head portion 32. As shown in FIG. 16C, the head portion 32 may include a distal surface 85 opposite the proximal surface 84 from which the neck portion 34 extends from. In some instances, the recess 82 may extend into the head portion 32 from the proximal surface 84 toward the distal surface 85, but does not extend to the distal surface 85. However, in other embodiments the recess 82 may extend to the distal surface 85.

The head portion 32 may also include a member extending across the recess 82 dividing the recess 82 into a first recess portion 82a on a first side of the member and a second recess portion 82b on a second side of the member. As shown in FIGS. 16A-16C, the member may be a pin 88 extending across the recess 82 or bridging across the recess 82. In some instances, the pin 88 may extend generally perpendicular to the recess 82 and/or the longitudinal axis of the housing 12. The pin 88 may be inserted through a hole 89 in the head portion 32 to position the pin 88 across the recess 82, for example.

A tether 80 may extend through a passage beneath the pin 88 defined by the recess 82 during delivery of the implantable device 10. For example, the tether 80 may be attached to the pin 88 and extend proximally from the pin 88 along an elongate shaft of a delivery device to a location accessible by a physician during implantation of the implantable device 10. Once the implantable device 10 has been properly implanted in the heart H, the tether 80 may be detached from the pin 88 and withdrawn from the patient.

It is noted that in other embodiments the tether 80 may be attached to the docking member 30 (such as through a passage in the docking member 30) and extend proximally from the docking member 30 along an elongate shaft of a delivery device to a location accessible by a physician during implantation of the implantable device 10. Similarly, once the implantable device 10 has been properly implanted in the heart H, the tether 80 may be detached from the docking member 30 and withdrawn from the patient

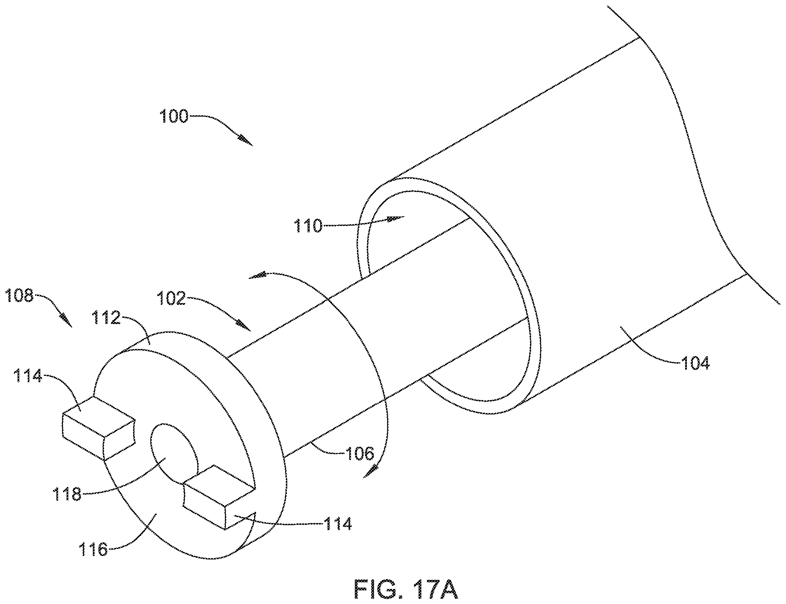

An exemplary delivery device 100 including a rotational driving instrument 102 and a delivery sheath 104 is illustrated in FIG. 17A. The rotational driving instrument 102 may include an elongate drive shaft 106 and a driver mechanism 108 at the distal end of the elongate drive shaft 106. The driver mechanism 108 may be configured for engagement with the head portion 32 of the docking member 30 to transfer rotational and/or longitudinal movement therebetween. The rotational driving instrument 102 may extend through a lumen 110 of the delivery sheath 104. The driving instrument 102 may be rotatable and longitudinally movable relative to the delivery sheath 104. The delivery sheath 104 may be sized such that the implantable device 10 may be positioned in a distal region of the lumen 110, with the driving instrument 102 engaged with a proximal portion of the implantable device 10 and extending proximally therefrom.

The driver mechanism 108 may include a pusher 112, such as a plate, located at the distal end of the elongate shaft 106 having a distal end surface 116 configured to engage the proximal surface 84 of the docking member 30. The driver mechanism 108 may also include one or more, or a plurality of protuberances, such as lugs 114, extending distally from the distal end surface 116 of the pusher 112, or otherwise arranged. For example, the driver mechanism 108 shown in FIG. 17A includes a first lug 114 and a second lug 114 spaced from the first lug 114 and extending in a distal direction from the distal end surface 116 of the pusher 112. The lug(s) 114 may be configured to engage in the recess 82 of the head portion 32 of the docking member 30.

The rotational driving instrument 102 may also include a lumen 118 extending therethrough. For example, the lumen 118 may extend through the elongate shaft 106 to an opening in the distal end surface 116 of the pusher 112 of the driver mechanism 108. The lumen 118 may be configured to receive the tether 80 therethrough such that the tether 80 may extend along the delivery device 100 to a proximal region of the delivery device 100 through the driving instrument 102. In other instances, the tether 80 may extend along the delivery device 100 through the lumen 110 of the delivery sheath 104 and external of the driving instrument 102, for example.

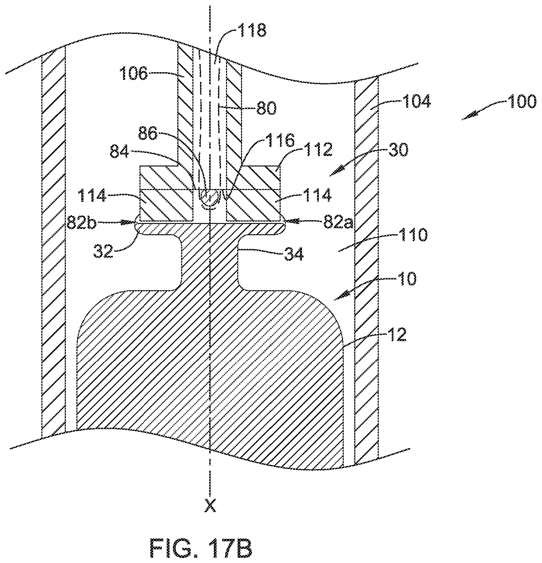

FIG. 17B illustrates an exemplary interaction between the delivery device 100 and the implantable device 10 with the docking member 30 shown in FIGS. 15A-15C during delivery and implantation of the implantable device 10 in a heart H, or other desired anatomy. As shown in FIG. 17B, the implantable device 10 may be positioned in the lumen 110 of the delivery sheath 104 with the driving instrument 102 engaged with the docking member 30. For instance, the distal end surface 116 of the pusher 112 may abut the proximal surface 84 of the head portion 32 of the docking member 30 while the first lug 114 is positioned in the first recess portion 82a on a first side of the member 86 and the second lug 114 is positioned in the second recess portion 82b on a second side of the member 86.

Accordingly, with the driver mechanism 108 engaged to the docking member 30, rotational movement of the driving instrument 102 may be transferred to the implantable device 10 to screw the helical fixation anchor 90 into a tissue wall and/or unscrew the helical fixation anchor 90 from a tissue wall.

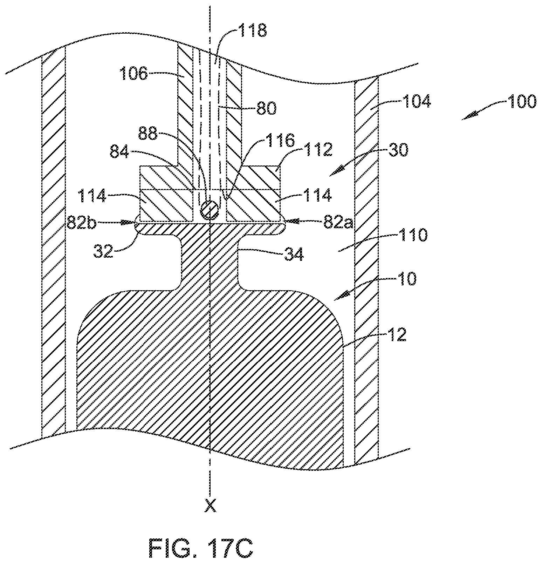

FIG. 17C illustrates an exemplary interaction between the delivery device 100 and the implantable device 10 with the docking member 30 shown in FIGS. 16A-16C during delivery and implantation of the implantable device 10 in a heart H, or other desired anatomy. In many respects the interaction may be similar to that described above regarding FIG. 17B. However, in FIG. 17C, the member extending across the recess 82 is shown as a pin 88. Accordingly, when the driver mechanism 108 is engaged to the docking member 30, the distal end surface 116 of the pusher 112 may abut the proximal surface 84 of the head portion 32 of the docking member 30 while the first lug 114 is positioned in the first recess portion 82a on a first side of the pin 88 and the second lug 114 is positioned in the second recess portion 82b on a second side of the pin 88.

Accordingly, with the driver mechanism 108 engaged to the docking member 30, rotational movement of the driving instrument 102 may be transferred to the implantable device 10 to screw the helical fixation anchor 90 into a tissue wall and/or unscrew the helical fixation anchor 90 from a tissue wall.

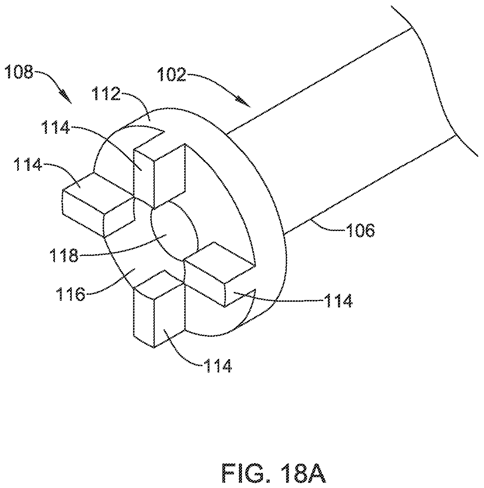

FIGS. 18A-18D illustrate alternative embodiments of a driver mechanism 108 for a rotational driving instrument 102 configured to mate with a docking member of an implantable device, such as one or more of the docking members 30 of the implantable device 10, described herein.

The driver mechanism 108 shown in FIGS. 18A-18C may be similar to the driver mechanism 108 shown in FIGS. 17A-17C. For example, the rotational driving instrument 102 may include an elongate drive shaft 106 and a driver mechanism 108 at the distal end of the elongate drive shaft 106. The driver mechanism 108 may be configured for engagement with the head portion 32 of the docking member 30 to transfer rotational and/or longitudinal movement therebetween. The rotational driving instrument 102 may extend through a lumen 110 of the delivery sheath 104 (shown in FIG. 17A).

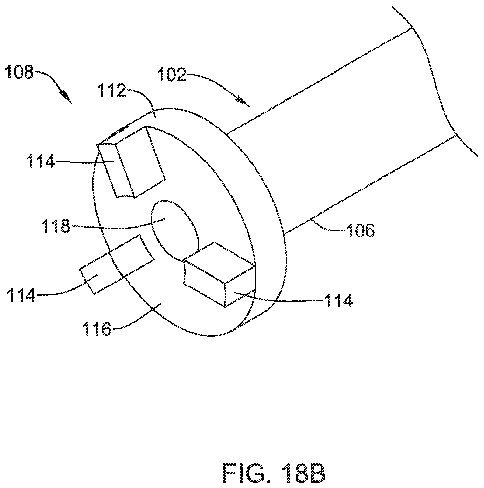

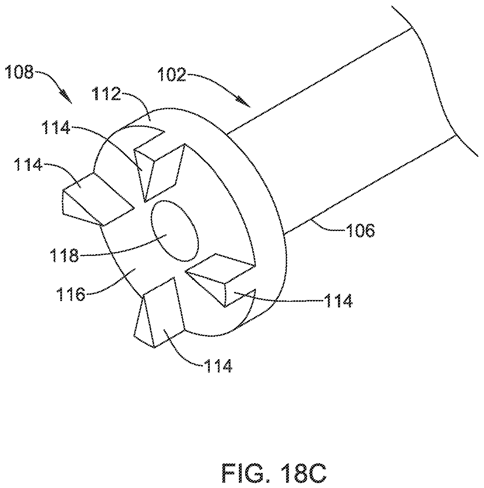

The driver mechanism 108 may include a pusher 112 having a distal end surface 116 configured to engage the proximal surface 84 of the docking member 30 and one or more, or a plurality of protuberances, such as lugs 114, extending distally from the distal end surface 116 of the pusher 112. For example, the driver mechanism 108 shown in FIG. 18A includes four equally spaced apart lugs 114, the driver mechanism 108 shown in FIG. 18B includes three equally spaced apart lugs 114, and the driver mechanism 108 shown in FIG. 18C includes four equally spaced apart lugs 114 extending in a distal direction from the distal end surface 116 of the pusher 112. The lug(s) 114 may be configured to extend into openings between adjacent spokes 70 of the docking members 30 described herein, and engage in the spokes 70 of the head portion 32 of the docking member 30 to transfer rotational torque therebetween.

The rotational driving instrument 102 may also include a lumen 118 extending through the elongate shaft 106 configured to receive the tether 80 therethrough such that the tether 80 may extend along the delivery device 100 to a proximal region of the delivery device 100 through the driving instrument 102. In other instances, the tether 80 may extend along the delivery device 100 through the lumen 110 of the delivery sheath 104 and external of the driving instrument 102, for example.

The shape, size, quantity and arrangement of the lugs 114 may be chosen to complement and mate with the shape, size, quantity and arrangement of spokes 70 of the docking member 30. For example, the lugs 114 shown in FIG. 18C, may be triangular or wedge shaped to fit between adjacent spokes 70 of the docking member 30 shown in FIGS. 8A, 9A, 10A or 14A. In other instances, the shape of the lugs 114 may be chosen to complement and mate with the spokes 70 shown in FIGS. 11A, 12A or 13A, for example.

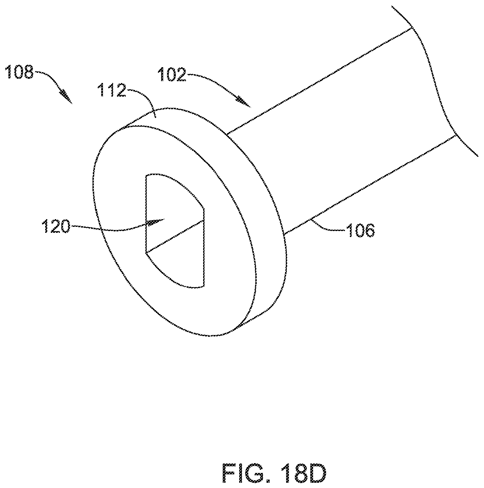

In other instances, as shown in FIG. 18D, the driver mechanism 108 of the rotational driving instrument 102 may include a socket 120 shaped, sized and configured to mate with the head portion 32 of the docking member 30 to transfer rotational torque therebetween. For example, as shown in FIG. 18D, the socket 120 may include opposing arcuate edges and opposing flat edges extending between the arcuate edges, configured to complement the shape and size of the docking member 30 shown in FIG. 4A. The flat edges of the socket 120 may engage the flat sides of the docking member 30 to transfer rotational torque therebetween. It is noted that in some instances, the socket 120 may include a single flat side for engagement with a flat side of the docking member 30.

In other instances, the socket 120 may have another shape, size and configuration to mate with the head portion 32 of another docking member 30. For example, the socket 120 may include a complementary shape, size and configuration to the head portion 32 of the docking member 30 shown in FIGS. 8A, 9A, 10A, 11A, 12A, 13A or 14A such that the head portion 32 of the docking member 30 fits into the socket 120. The socket 120 may include at least one edge or surface configured to engage a surface of the head portion 32 of the docking member 30 to transfer rotational torque therebetween.

Those skilled in the art will recognize that aspects of the present disclosure may be manifested in a variety of forms other than the specific embodiments described and contemplated herein. Accordingly, departure in form and detail may be made without departing from the scope of the present disclosure as described in the appended claims.

## Claims

1. A system for implanting an implantable cardiac pacing device, the system including:
an implantable leadless cardiac pacing device (10) comprising:
a housing (12) having a proximal end and a distal end;
an electrode (20) positioned proximate the distal end of the housing configured to be positioned adjacent cardiac tissue; and
a docking member (30) extending from the proximal end of the housing along a longitudinal axis of the housing, the docking member configured to facilitate retrieval of the implantable leadless cardiac pacing device;
the docking member including a head portion (32) and a neck portion (34) extending between the housing and the head portion;
the head portion having a radial dimension from the longitudinal axis and the neck portion having a radial dimension from the longitudinal axis less than the radial dimension of the head portion;
the head portion including a recess (82) extending into the head portion from a proximal surface of the head portion for receiving a rotational driving instrument; and
the system further including
a delivery device including an elongate shaft and a driver mechanism at a distal end of the elongate shaft, the driver mechanism configured for engagement with the head portion of the docking member;
wherein the driver mechanism includes a first lug configured to engage the recess extending into the head portion to transfer rotational motion from the driver mechanism to the implantable cardiac pacing device.

2. The implantable leadless cardiac pacing device of claim 1, further comprising a member extending across the recess; and wherein the member optionally extends generally perpendicular to the recess, in particular, wherein each of the member and the recess optionally extend generally perpendicular to the longitudinal axis of the housing.

3. The implantable leadless cardiac pacing device of claim 2, wherein the member divides the recess into a first portion on a first side of the member and a second portion on a second side of the member.

4. The implantable leadless cardiac pacing device of claim 2, further comprising a tether attached to the member; and wherein the tether optionally extends along the elongate shaft of the delivery device, in particular, wherein the tether optionally extends through a lumen of the elongate shaft of the delivery device.

5. The implantable leadless cardiac pacing device of claim 1, wherein the recess extends across the head portion from a first side of the head portion to a second side of the head portion.

6. The implantable leadless cardiac pacing device of claim 1, wherein the head portion is a knob having a first diameter and the neck portion has a second diameter less than the first diameter.

7. The implantable leadless cardiac pacing device of claim 1, wherein the head portion has a distal surface opposite the proximal surface from which the neck portion extends from, wherein the recess does not extend to the distal surface.

8. The system of claim 1, wherein the driver mechanism includes a second lug configured to engage the recess of the head portion.

9. The system of claim 2 and 8, wherein the first lug is positionable in a first portion of the recess on a first side of the member and the second lug is positionable in a second portion of the recess on a second side of the member.

10. The system of claim 8, wherein the first and second lugs extend from an end surface of the driver mechanism, wherein the end surface is configured to abut the proximal surface of the head portion.

## Patentansprüche

1. System zum Implantieren einer implantierbaren Herzschrittmachervorrichtung, wobei das System umfasst:
eine implantierbare kabellose Herzschrittmachervorrichtung (10), umfassend:
ein Gehäuse (12) mit einem proximalen Ende und einem distalen Ende;
eine Elektrode (20), die benachbart zum distalen Ende des Gehäuses angeordnet ist, das eingerichtet ist, dem Herzgewebe benachbart positioniert zu sein; und
ein Andockelement (30), das sich vom proximalen Ende des Gehäuses entlang einer Längsachse des Gehäuses erstreckt, wobei das Andockelement eingerichtet ist, das Zurückholen der implantierbaren kabellosen Herzschrittmachervorrichtung zu ermöglichen;
wobei das Andockelement einen Kopfabschnitt (32) und einen Halsabschnitt (34), der sich zwischen dem Gehäuse und dem Kopfabschnitt erstreckt, aufweist;
wobei der Kopfabschnitt eine radiale Abmessung von der Längsachse aufweist und der Halsabschnitt eine radiale Abmessung von der Längsachse aufweist, die geringer ist als die radiale Abmessung des Kopfabschnitts;
der Kopfabschnitt eine Aussparung (82) enthält, die sich in den Kopfabschnitt von einer proximalen Oberfläche des Kopfabschnitts erstreckt, um ein Rotationsantriebsinstrument aufzunehmen; und
das System ferner umfasst:
eine Einbringvorrichtung enthaltend einen länglichen Schaft und ein Antriebsmechanismus an einem distalen Ende des länglichen Schafts, wobei der Antriebsmechanismus für den Eingriff mit dem Kopfabschnitt des Andockelements eingerichtet ist;
wobei der Antriebsmechanismus eine erste Nase aufweist, die eingerichtet ist, in die sich in den Kopfabschnitt erstreckende Aussparung einzugreifen, um eine Drehbewegung vom Antriebsmechanismus auf die implantierbare Herzschrittmachervorrichtung zu übertragen.

2. Implantierbare kabellose Herzschrittmachervorrichtung nach Anspruch 1, ferner umfassend ein Element, das sich über die Aussparung erstreckt; und wobei sich das Element optional im Allgemeinen senkrecht zu der Aussparung erstreckt, insbesondere, wobei sich jedes von dem Element und der Aussparung optional im Allgemeinen senkrecht zu der Längsachse des Gehäuses erstrecken.

3. Implantierbare kabellose Herzschrittmachervorrichtung nach Anspruch 2, wobei das Element die Aussparung in einen ersten Abschnitt auf einer ersten Seite des Elements und einen zweiten Abschnitt auf einer zweiten Seite des Elements unterteilt.

4. Implantierbare kabellose Herzschrittmachervorrichtung nach Anspruch 2, ferner umfassend eine an dem Element befestigte Schlaufe; und wobei sich die Schlaufe optional entlang des länglichen Schafts der Einbringvorrichtung erstreckt, insbesondere wobei sich die Schlaufe optional durch ein Lumen des länglichen Schafts der Einbringvorrichtung erstreckt.

5. Implantierbare kabellose Herzschrittmachervorrichtung nach Anspruch 1, wobei sich die Aussparung quer über den Kopfabschnitt von einer ersten Seite des Kopfabschnitts zu einer zweiten Seite des Kopfabschnitts erstreckt.

6. Implantierbare kabellose Herzschrittmachervorrichtung nach Anspruch 1, wobei der Kopfabschnitt ein Knauf mit einem ersten Durchmesser ist und der Halsabschnitt einen zweiten Durchmesser aufweist, der kleiner als der erste Durchmesser ist.

7. Implantierbare kabellose Herzschrittmachervorrichtung nach Anspruch 1, wobei der Kopfabschnitt eine distale Oberfläche aufweist, die der proximalen Oberfläche, von der sich der Halsabschnitt erstreckt, gegenüberliegt, wobei sich die Aussparung nicht bis zur distalen Oberfläche erstreckt.

8. System nach Anspruch 1, wobei der Antriebsmechanismus eine zweite Nase aufweist, die eingerichtet ist, in die Aussparung des Kopfabschnitts einzugreifen.

9. System nach Anspruch 2 und 8, wobei die erste Nase in einem ersten Abschnitt der Aussparung auf einer ersten Seite des Elements positionierbar ist und die zweite Nase in einem zweiten Abschnitt der Aussparung auf einer zweiten Seite des Elements positionierbar ist.

10. System nach Anspruch 8, wobei die erste und die zweite Nase sich von einer Endfläche des Antriebsmechanismus erstrecken, wobei die Endfläche eingerichtet ist, an der proximalen Fläche des Kopfabschnitts anzuliegen.

## Revendications

1. Système pour implanter un dispositif de stimulation cardiaque implantable, le système incluant :
un dispositif de stimulation cardiaque sans fil implantable (10) qui comprend :
un boîtier (12) qui comporte une extrémité proximale et une extrémité distale ;
une électrode (20) qui est positionnée à proximité de l'extrémité distale du boîtier et qui est configurée pour être positionnée de telle sorte qu'elle soit adjacente au tissu cardiaque ; et
un élément d'accrochage (30) qui est étendu depuis l'extrémité proximale du boîtier le long d'un axe longitudinal du boîtier, l'élément d'accrochage étant configuré pour faciliter la récupération du dispositif de stimulation cardiaque sans fil implantable ;
l'élément d'accrochage incluant une partie de tête (32) et une partie de col (34) qui est étendue entre le boîtier et la partie de tête ;
la partie de tête présentant une dimension radiale par rapport à l'axe longitudinal et la partie de col présentant une dimension radiale par rapport à l'axe longitudinal qui est plus petite que la dimension radiale de la partie de tête ;
la partie de tête incluant un évidement (82) qui est étendu à l'intérieur de la partie de tête depuis une surface proximale de la partie de tête pour recevoir un instrument d'entraînement en rotation ; et
le système incluant en outre :
un dispositif de mise en place qui inclut un arbre allongé et un mécanisme de dispositif d'entraînement au niveau d'une extrémité distale de l'arbre allongé, le mécanisme de dispositif d'entraînement étant configuré pour être engagé avec la partie de tête de l'élément d'accrochage ;
dans lequel le mécanisme de dispositif d'entraînement inclut une première languette qui est configurée pour être engagée avec l'évidement qui est étendu à l'intérieur de la partie de tête afin de transférer un mouvement de rotation depuis le mécanisme de dispositif d'entraînement au dispositif de stimulation cardiaque implantable.

2. Dispositif de stimulation cardiaque sans fil implantable selon la revendication 1, comprenant en outre un élément qui est étendu au travers de l'évidement ; et dans lequel l'élément est étendu en option de façon générale perpendiculairement à l'évidement, en particulier dans lequel chaque partie constitutive prise parmi l'élément et l'évidement est étendue en option de façon générale perpendiculairement à l'axe longitudinal du boîtier.

3. Dispositif de stimulation cardiaque sans fil implantable selon la revendication 2, dans lequel l'élément divise l'évidement selon une première partie sur un premier côté de l'élément et selon une seconde partie sur un second côté de l'élément.

4. Dispositif de stimulation cardiaque sans fil implantable selon la revendication 2, comprenant en outre une attache qui est liée à l'élément ; et dans lequel l'attache est étendue en option le long de l'arbre allongé du dispositif de mise en place, en particulier dans lequel l'attache est étendue en option au travers d'une lumière de l'arbre allongé du dispositif de mise en place.

5. Dispositif de stimulation cardiaque sans fil implantable selon la revendication 1, dans lequel l'évidement est étendu au travers de la partie de tête depuis un premier côté de la partie de tête jusqu'à un second côté de la partie de tête.

6. Dispositif de stimulation cardiaque sans fil implantable selon la revendication 1, dans lequel la partie de tête est un bouton qui présente un premier diamètre et la partie de col présente un second diamètre qui est plus petit que le premier diamètre.

7. Dispositif de stimulation cardiaque sans fil implantable selon la revendication 1, dans lequel la partie de tête comporte une surface distale qui est opposée à la surface proximale depuis laquelle la partie de col est étendue, et dans lequel l'évidement n'est pas étendu jusqu'à la surface distale.

8. Système selon la revendication 1, dans lequel le mécanisme de dispositif d'entraînement inclut une seconde languette qui est configurée pour engager l'évidement de la partie de tête.

9. Système selon les revendications 2 et 8, dans lequel la première languette peut être positionnée à l'intérieur d'une première partie de l'évidement sur un premier côté de l'élément et la seconde languette peut être positionnée à l'intérieur d'une seconde partie de l'évidement sur un second côté de l'élément.

10. Système selon la revendication 8, dans lequel les première et seconde languettes sont étendues depuis une surface d'extrémité du mécanisme de dispositif d'entraînement, et dans lequel la surface d'extrémité est configurée pour venir en butée contre la surface proximale de la partie de tête.
